# EUROPEAN PATENT APPLICATION

(11) **EP 1 277 728 A1**
(43) Date of publication of application: **22.01.2003**
(21) Application number: 01117355.6
(22) Date of filing: 18.07.2001
(51) Int. Cl.: C07C 229/16, A61K 49/06

(54) **Novel TTDA derivatives and their use as gadolinium chelators for MRI imaging**

(71) Applicant: University of Lausanne, 1005 Lausanne (CH)
(72) Inventor: Merbach, André Prof., 1009 Pully (CH); Toth, Eva Jakab, 1022 Chavannes (CH); Ruloff, Robert, 1022 Chavannes (CH); Laus, Sabrina, 1004 Lausanne (CH)
(74) Representative: Kiliaridis, Constantin

(57) **Abstract**

The invention relates to derivatives of TTDA (also known as EPTPA) when one of the hydrogen atom is substituted by a chemically, reactive group suitable for coupling with a macromolecule, complexes comprising the ligands and a metal ion, and adducts of these metal complexes and a macromolecule. Also described are pharmaceutical compositions, methods of making and using the ligand-metal complexes and the macromolecular adducts for enchancement of diagnostic imaging, especially MRI.

## Description

### FIELD OF THE INVENTION

The invention relates to a novel class of ligands, complexes comprising such ligands and a metal ion, and adducts of these metal complexes and a macromolecule. Pharmaceutical compositions and methods of making and using the ligand-metal complexes are also described. The invention also relates to the use of macromolecular adducts for enhancement of diagnostic imaging.

### BACKGROUND OF THE INVENTION

X-rays have long been used to produce images of human and non-human animal tissue, e.g., the internal organs of a patient. Typically, the patient is positioned between a source of X-rays and a film sensitive to the rays. Where organs interfere with the passage of the rays, the film is less exposed and the resulting developed film is indicative of the state of the organ. More recently, nuclear magnetic resonance (NMR) has been applied in medical imaging as magnetic resonance imaging (MRI). MRI avoids the harmful effects sometimes associated with exposure to X-rays.

To improve the quality of such diagnostic images, patients are often given image enhancers, or contrast agents, prior to image acquisition. For example, in X-ray diagnostics, increased contrast of internal organs such as the kidneys, the urinary tract, the digestive tract and the vascular system of the heart may be obtained by administering a radiopaque agent to the patient. In conventional proton MRI diagnostics, increased contrast of internal organs and tissues may be obtained by administering compositions containing paramagnetic metal species, which increase the relaxation rate of surrounding protons. In ultrasound diagnostics, improved contrast is obtained by administering compositions having acoustic impedances which are different than that of blood or other tissues.

Many interesting contrast agents are those in which organic acid ligands are coordinated to a metal atom or cation. The nature of substituents of the ligand, or complexing agent, can have a significant impact on tissue specificity of the contrast agent. For example, hydrophilic complexes tend to concentrate in the interstitial fluids, whereas lipophilic complexes tend to associate with cells. Thus, differences in hydrophilicity can lead to different applications of the compounds. The metal-ligand complex may be charged or neutral, and the charge may be altered to affect solubility.

If not all of the hydrogen atoms of an acidic ligand are substituted by the central (metal) ion (or central ions), it may be advantageous to increase the solubility of the complex salt by substituting the remaining hydrogen atoms with cations of inorganic and/or organic bases or amino acids. For example, the hydroxides, carbonates or bicarbonates of sodium, potassium or lithium are suitable inorganic cations. Suitable cations of organic bases include, among others, those of primary, secondary or tertiary amines, for example, ethanolamine, diethanolamine, morpholine, glucamine, N,N-dimethylglucamine or especially N-methylglucamine. Lysines, arginines or ornithines are suitable cations of amino acids, as generally are those of other basic naturally occurring such acids. If the complex salts contain several free acid groups, it is then often advantageous to produce neutral mixed salts which contain both inorganic and organic cations as counterions.

The complexing agents can also be coupled as conjugates with biomolecules that are known to concentrate in a particular organ or the part of an organ to be examined. These biomolecules include, for example, hormones (*e.g.*, insulin), prostaglandins, steroid hormones, amino sugars, peptides, proteins, lipids, etc. Conjugates with albumins *(e.g.,* human serum albumin) or antibodies, (*e.g.*, monoclonal antibodies specific for tumor associated antigens or proteins such as myosin, etc.) are especially notable. The diagnostic agents formed therefrom can be used, *e.g.*, to diagnose tumors and mycoardial infarctions. Conjugates with liposomes, or inclusion of salts of the contrast agent in liposomes, (*e.g.* unilamellar or multilamellar phosphatidylcholine-cholesterol vesicles) are suitable for liver imaging.

For X-ray diagnosis, the central ion in the contrasting agent is derived from an element with a high atomic number in order to promote sufficient absorption of X-rays. Diagnostic media containing a physiologically well tolerated complex salt containing a central ion chosen from elements with atomic numbers of 57 to 83 are suitable for this purpose. These include, for example, lanthanum(III), and other di- and tri-valent ions of the lanthanide group, gold(III), lead(II) or, especially, bismuth(III).

Chemical compounds with paramagnetic central ions are useful for MRI imaging. Two important MRI imaging parameters are spin-lattice (T1) and spin-spin and spin-echo (T2) relaxation times. The relaxation phenomena are essentially mechanisms whereby the initially imparted radio-frequency energy is dissipated to the surrounding environment. Thus, relaxation times are influenced by the environment of the nuclei, *(e.g.,* viscosity, and temperature). The rate of energy loss, or relaxation, can also be influenced by neighboring paramagnetic nuclei. As such, chemical compounds incorporating paramagnetic nuclei may substantially alter the T1 and T2 values for nearby protons.

Nuclei which are useful in MRI contrasting agents include organic free radicals or transition or lanthanide metals which have from one to seven unpaired electrons. In general, paramagnetic species such as ions of elements with atomic numbers of 21 to 29, 42 to 44 and 58 to 70 are effective. Examples of suitable ions include chromium(III), manganese(II), manganese(III), iron(II), iron(III), cobalt(II), nickel(II), copper(II), praseodymium(III), neodymium(III), samarium(III), and ytterbium(III). Because of their very strong magnetic moments, gadolinium(III), terbium(III), dysprosium(III), holmium(III), and erbium(III) are preferred. Gadolinium(III) ions have been particularly useful as MRI contrasting agents.

Typically, paramagnetic ions have been administered in the form of complexes with organic complexing agents. A necessary prerequisite of any ligand that binds a metal to form a contrast agent is that the resulting contrast agent be stable so as to prevent the loss of the metal and its subsequent accumulation in the body. Such complexes provide the paramagnetic ions in a soluble, non-toxic form, and facilitate their rapid clearance from the body following the imaging procedure.

An example of a contrasting agent recognized in the art is gadolinium(III) with diethylenetriamine-pentaacetic acid ("DTPA"). Paramagnetic ions, such as gadolinium(III), have been found to form strong complexes with DTPA, ethylenediamine-tetra acetic acid ("EDTA"), and with tetra aza-cyclododecane-N,N',N",N'''-tetra acetic acid ("DOTA"). In addition to their use as contrast agents, lanthanide(III) poly-aminocarboxylates are also widely used as luminescent probes in fluoroimmunoassays.

Because the relaxivity of sphere-shaped molecules increases approximately linearly with molecular weight, and the relaxation of trivalent gadolinium, Gd(III),- containing contrast agents is mainly limited by their fast rotational motion, the incorporation of Gd chelates in large structures slows their rotational motion and increases relaxivity properties. Such structures can include polymers (Desser, *et al.*, *J*. *Magn. Reson. Imaging* 1994, *4*, 467); dendrimers (Tacke, *et al.*, *J. Magn. Reson. Imaging,* 1997, *7*, 678); proteins (Lauffer and Brady, *J*. *Magn. Reson. Imaging*, 1985, 3, 11) and micelles (André, *et al.*, *Chem. Eur. J.*, 1999, *5*, 2977).

Several approaches for making contrasting agents utilize supramolecular chemistry (Aime, *et al.*, *Chem. Eur. J.*, 1999, *5*, 1253; Jacques, *et al.*, *Coord. Chem. Rev.*, 1999, *185*, 451) and self-organization (André, *et al.*, *Chem. Eur. J.*, 1999, *5*, 2977). For example, poly-β-cyclodextrins have been used to bind gadolinium poly-aminocarboxylate chelates that contain a lipophilic phenyl tail (Aime, *et al.*, *Chem. Eur. J.*, 1999, *5*, 1253), and polymetallic gadolinium-containing structures have been self-assembled through secondary recognition involving octahedral transition metals (Jacques, *et al.*, *Coord. Chem. Rev.*, 1999, *185,* 451).

One important approach to developing efficient MRI contrast agents can involve the association of monomers into reversible supramolecular structures. This is accomplished by exploiting short distance interactions, (*i.e.*, hydrogen bonds, aromatic π-stacking and van der Waal's interactions), which can be used for molecular recognition based upon complementary size, shape and chemical functionalities. The monomers used for the formation of supramolecular contrast agents must be rigid enough to ensure good intermolecular contact between interacting surfaces and also must overcome the loss of translational entropy of the monomers upon aggregation.

Contrast agents can be plagued by the *in vivo* release of free metal ions from the complex, which can result in metal toxicity subject. The toxicity of paramagnetic metal complexes can be affected by the nature of the complexing ligands. Principal factors involved in the design of ligands for paramagnetic metal complexes include the thermodynamic stability constant of the metal-ligand complex (the affinity of the totally unprotonated ligand for the metal); the conditional stability constant (which is pH dependent and is important when considering stability under physiological pH); the selectivity of the ligand for the paramagnetic metal over other endogenous metal ions (e.g., zinc, iron, magnesium and calcium); and the structural features that make in vivo transmetallation reactions much slower than the clearance rate of the complex.

### SUMMARY OF THE INVENTION

The invention provides a novel class of ligands, complexes comprising such ligands and a metal ion, and adducts in which these metal complexes are coupled (covalently or non-covalently) to a macromolecule. Pharmaceutical compositions and methods of making and using the ligand-metal complex and the macromolecular adducts for enhancement of diagnostic imaging are also described.

These metal-ligand complexes and their macromolecular adducts are useful as MRI contrast agents, diagnostic agents in X-ray, ultrasound or scintigraphic image analysis, as radiotherapy agents, and as luminescent probes. Because the macromolecular adducts have an unexpectedly high relaxivity, much less of the complex is required to be administered to the subject relative to commonly used image enhancing agents.

The compounds of the invention are chelating ligands which provide optimized water exchange rates of their Gd(III) complexes.

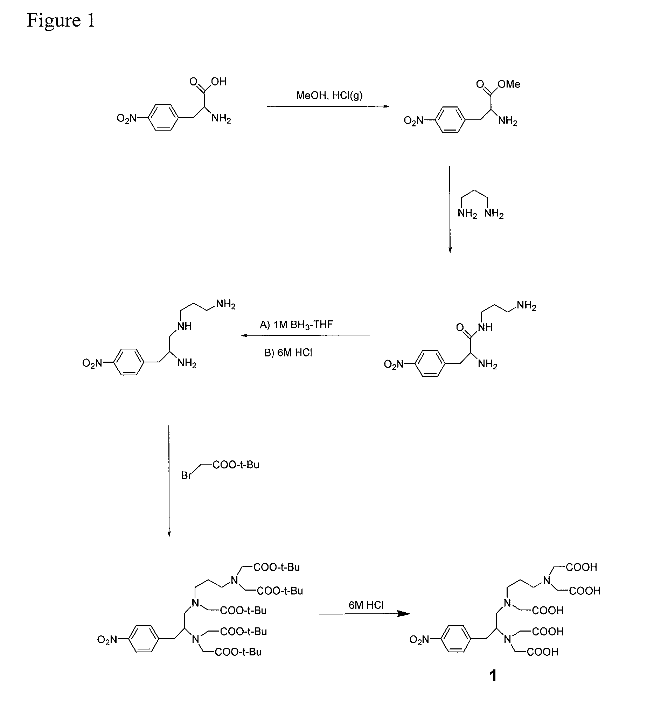

For example, the ligand EPTPA-bz-NO₂ (Formula 1) has been synthesized as described in Figure 1. The chelate contains a group which can function as a linker to couple the ligand to macromolecules according to well-documented procedures. One example of such a group is an isothiocyanate group. The macromolecules can be biological molecules such as proteins, carbohydrates, lipids, or any synthetic, biocompatible materials.

The invention also provides a method of magnetic resonance imaging by administering to a human or non-human animal subject a contrast medium that includes a physiologically compatible paramagnetic metal complex of the herein described ligands and a non-toxic, pharmaceutically acceptable carrier, adjuvant or vehicle in an amount sufficient to allow for the generation of a magnetic resonance image of at least a part of the subject.

Further according to the invention, a method of diagnostic imaging is provided which comprises administering to a human or non-human animal subject a diagnostic agent comprising a physiologically compatible heavy metal complex of the present invention and a non- toxic, pharmaceutically acceptable carrier, adjuvant or vehicle, and generating an X-ray, ultrasound or scintigraphic image of at least a part of the subject.

Further according to the invention, a method of radiotherapy practiced on a human or non-human animal subject is provided which comprises administering to the subject a radioactive agent comprising a physiologically compatible radioactive metal complex of the present invention and a non-toxic, pharmaceutically acceptable carrier, adjuvant or vehicle.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and are not intended to be limiting.

Other features and advantages of the invention will be apparent from the following detailed description and claims.

### BRIEF DESCRIPTION OF THE DRAWINGS

- FIG. 1: shows a scheme for the synthesis of one example of the class of ligands of the invention.
- FIG. 2: shows the NMRD profiles of Gd(EPTPA-bz-NO2) at 37 C (bottom curve) and 25 C (top curve).
- FIG. 3: shows the ¹⁷O NMR, NMRD and EPR experimental data (points) and the fitted curves (lines) for Gd(EPTPA-bz-NO2). 3A: reduced transverse (i = 2) and longitudinal (i=1) ¹⁷O relaxation rates (B = 9.4 T), 3B: reduced ¹⁷O chemical shifts (B = 9.4 T). 3C: NMRD profiles. 3D: transverse electron spin relaxation rates at 0.34 T, as measured by EPR.

### DETAILED DESCRIPTION OF THE INVENTION

The present invention provides a novel class of ligands falling within Formula III, as set forth below: 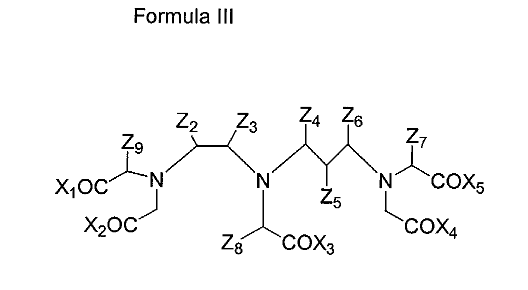 or a base or acid addition salt, hydrate, ester, solvate, prodrug, metabolite, stereoisomer, or mixture thereof, wherein at least one of Z₂ Z_{3,} Z_{4,} Z_{5,} Z_{6,} Z_{7,} Z_{8,} or Z₉ is a functionality suitable for coupling with a biocompatible macromolecule and the remaining Z₂ Z_{3,} Z_{4,} Z_{5,} Z_{6,} Z_{7,} Z_{8,} or Z₉ are non-coordinating substituents, X_{1,} X_{2,} X_{3,} X₄ and X₅ are independently OH or NR₂ wherein each R is a non-coordinating substituent, and at least two of X_{1,} X_{2,} X_{3,} X₄ and X₅ are OH.

Non-coordinating substituents include those that do not coordinate to a metal, such as alkyl groups and hydrogen atoms.

In some embodiments, the invention includes compounds of Formula II or Formula I:

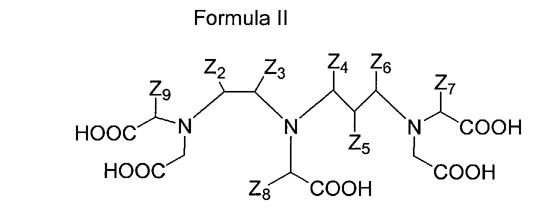

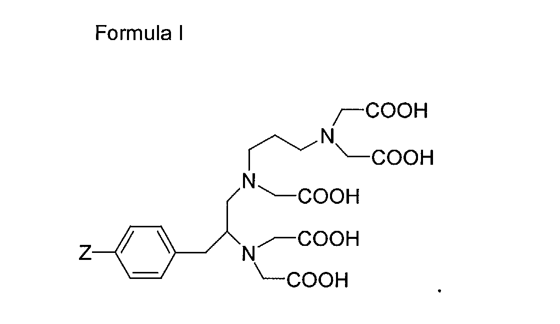

Preferably, the base or acid addition salt is a pharmaceutically acceptable salt. Salts encompassed within the term "pharmaceutically acceptable salt" are non-toxic salts of the compounds of this invention which are generally prepared by reacting an acidic complex with physiologically biocompatible cations of organic and/or inorganic bases or amino acids to produce "pharmaceutically-acceptable acid addition salts" of the compounds described herein. These compounds retain the biological effectiveness and properties of the free complexes. For example, the lithium ion, the potassium ion and especially the sodium ion are suitable inorganic cations. Suitable cations of organic bases include, among others, those of primary, secondary or tertiary amines, for example, ethanolamine, diethanolamine, morpholine, glucamine, N,N-dimethylglucamine or especially N-methylglucamine. Lysines, arginines or ornithines are suitable cations of amino acids, as generally are those of other basic naturally occurring such acids.

The metal atoms or cations, M, which are suitable for use in the complexes of the invention as MRI contrast agents are paramagnetic metals having atomic numbers 21-29, 42-44 and 57-71. The complexes for use as MRI contrast agents are those wherein the preferred metal is Eu, Gd, Dy, Ho, Cr, Mn or Fe, more preferably Mn(II)or Fe(III), and most preferably Gd(III).

The metal atoms or cations which are suitable for use in the complexes of the invention as X-ray or ultrasound contrast agents are heavy metals having atomic numbers 20-32, 42-44, 49 and 57-83. The complexes for use as X-ray or ultrasound contrast agents are those wherein the preferred metal is a non-radioactive metal having atomic numbers 42-44, 49 and 57-83, most preferably Gd, Dy or Yb.

The metal atoms or cations of the complexes of the invention which are suitable for use in scintigraphic and radiotherapy are radioactive metals of any conventional complexable radioactive metal isotope, preferably those having atomic numbers 20-32, 42-44, 49 and 57-83. In scintigraphy, the most preferred metals are ^{99m} Tc or ¹¹¹In. In radiotherapy, the most preferred metals are ¹⁵³Sm, ⁶⁷Cu or ⁹⁰Y.

The metal atom or cations which are suitable for use as luminescence enhancers include, *e.g.*, Eu and Tb.

Interaction of the H₅L ligand with Ln³⁺ ions (Ln = Lanthanides, *e.g.*, Lanthanum (La), Europium (Eu), Lutetium (Lu), Gadolinium (Gd), and Terbium (Tb)) in dilute aqueous solution creates complexes with metal:ligand stoichiometry of 1:1. The 1:1 lanthanide complexes of the present invention display high thermodynamic stability under physiological conditions.

The compounds of the invention, including *e.g.,* those of Formula I, Formula II and Formula III, can be used to enhance images produced by method of magnetic resonance imaging. In one embodiment, a contrast medium made from a physiologically compatible complex of the invention and a nontoxic pharmaceutically acceptable carrier, adjuvant or vehicle is administered to a human or non-human animal (subject); and a magnetic resonance image is generated of at least a part of the subject. The compounds can similarly be used to enhance images produced by X-ray, ultrasound or scintigraphic imaging of a subject.

The methods of diagnostic analysis of the present invention involve administering the compounds of the invention to a human or non-human animal subject or host, in an amount sufficient to effect the desired contrast (or shift) and then subjecting the host to diagnostic analysis. Preferably diagnostic analysis is MRI analysis. Further, the complexes of the present invention are useful in diagnostic analysis by X-ray image analysis, ultrasonic analysis or scintigraphic analysis. While described primarily as contrast enhancing agents, the complexes of the invention can act as MRI shift reagents and such use is contemplated by the methods herein.

The complexes of the invention used as contrast enhancing agents are administered in an amount sufficient to effect the desired contrast. For MRI, this amount is an MRI signal effecting amount of the complex, i.e. any amount of said complex that will alter the spin-lattice (T1) or spin-spin or spin-echo (T2) relaxation times of an MRI signal. For a shift reagent, a sufficient amount of said complex will selectively shift the spectral position of a resonance nucleus relative to other similar nuclei. This alteration is effected in a manner in order to enhance the signals received from the subject under analysis either by reducing the aforementioned relaxation times or by increasing them with respect to an area of the host or the host per se which has had the complex administered to it. In another embodiment, the MRI signal effecting amount of the complex is that amount which in addition to changing the relaxation times of the MRI signals in the host, will also change such relaxation times sufficiently so that sharper lines of definition or higher contrast is obtained between those parts of the host that have and have not been administered the complex.

A detailed discussion of theoretical considerations for selecting the appropriate parameters for MRI diagnostic analysis is provided in U.S. Pat. No. 4,749,560, incorporated herein by reference. X-ray image analysis, ultrasonic diagnosis, scintigraphic image analysis and radiotherapy utilizing the complexes of the invention are all conducted in accordance with well-established techniques known to those of ordinary skill in the art.

The complexes of the invention may be administered to a host as a pharmaceutical composition in a contrast-enhancing amount. The pharmaceutical compositions contain a contrast-enhancing dosage of the contrast agents according to the invention together with a nontoxic pharmaceutically acceptable carrier, adjuvant or vehicle. The compositions can be administered by well-known routes including oral, intravenous, intramuscular, intranasal, intradermal, subcutaneous, parenteral, enteral and the like. Depending on the route of administration, the pharmaceutical composition may require protective coatings.

The pharmaceutical forms suitable for injectable use includes sterile solutions, suspensions, emulsions syrups or dispersions in oily or aqueous media and sterile powders for the extemporaneous preparation of sterile injectable solutions or dispersions. In all cases the ultimate solution form must be sterile and fluid. Typical carriers include a solvent or dispersion medium containing, for example, water, buffered aqueous solutions (i.e. biocompatable buffers), ethanol, polyol (glycerol, propylene glycol, polyethylene glycol, and the like), suitable mixtures thereof, surfactants or vegetable oils. Sterilization can be accomplished by any art recognized technique, including but not limited to, addition of antibacterial or antifungal agents, for example, paraben, chlorobutanol, phenol, sorbic acid, thimerosal, and the like. Further, isotonic agents, such as sugars or sodium chloride may be incorporated in the subject compositions.

Production of sterile injectable solutions containing the subject contrast agent is accomplished by incorporating these agents in the required amount in the appropriate solvent with various ingredients enumerated above, as required, followed by sterilization, preferably filter sterilization. To obtain a sterile powder, the above solutions are vacuum-dried or freeze-dried as necessary.

Solid dosage forms for oral administration may include capsules, tablets, pills, powders, granules and gels. In such solid dosage forms, the active compound may be admixed with at least one inert diluent such as sucrose, lactose or starch. Such dosage forms may also comprise, as in normal practice, additional substances other than inert diluent, e.g. lubricating agents such as magnesium stearate. In the case of capsules, tablets and pills, the dosage forms may also comprise buffering agents. Tablets and pills can additionally be prepared with enteric coatings. Liquid dosage forms for oral administration may include pharmaceutically acceptable emulsions, solutions, suspensions, syrups, and elixirs containing inert diluent commonly used in the art, such as water. Such compositions may also comprise adjuvants, such as wetting agents, emulsifying and suspending agents, and sweetening, flavoring, and perfuming agents.

The contrast agents of the inventions are thus compounded for convenient and effective administration in pharmaceutically effective amounts with a suitable pharmaceutically acceptable carrier, adjuvant or vehicle in a dosage which effects contrast enhancement. These amounts are preferably about 1 µmol to 1 mol of the contrast agent per liter and/or administered in doses of about 0.0001 to 5 mmol/kg body weight. Preferred compositions provide effective-dosages of contrast agents in the range of about 0.001-5 mmol/kg for MRI diagnostics, preferably about 0.005-0.5 mmol/kg; in the range of about 0.1-5 mmol/kg for X-ray diagnostics; and in the range of about 0.1-5 mmol/kg for ultrasound diagnostics. For scintigraphic diagnostics, the dose of the contrast agent should generally be lower than for MRI diagnostics. For radiotherapy, conventional doses known to those of ordinary skill in the art can be used.

As used herein, a pharmaceutically acceptable carrier, adjuvant or vehicle includes any and all solvents, dispersion media, coatings, antibacterial and antifungal agents, isotonic agents, and the like. The use of such media and agents are well known in the art.

In some embodiments, ligand of Formula I is coupled to a biological molecule prior to formation of the metal- ligand complex. In other embodiments, a ligand of Formula I is coupled to a biological molecule after formation of the metal-ligand complex has been accomplished. These conjugates are particularly useful as image enhancing agents. Useful biological molecules are those known to concentrate in a particular organ or the part of an organ to be examined. These biomolecules include, for example, hormones (*e.g.*, insulin), prostaglandins, steroid hormones, amino sugars, peptides, proteins, lipids, etc. Conjugates with albumins *(e.g.,* human serum albumin) or antibodies, (*e.g.*, monoclonal antibodies specific for tumor associated antigens or proteins such as myosin, etc.) are especially notable. The diagnostic agents formed therefrom are suitable, for example, for use in tumor and infarct diagnosis. Conjugates with liposomes, or inclusion of salts of the contrast agent in liposomes, (*e.g*. unilamellar or multilamellar phosphatidylcholine-cholesterol vesicles) are suitable for liver imaging. The use of these complexes will allow tissue- or organ-specific diagnostic analysis of a subject. For example, the contrast enhancing agents can exhibit organ and tissue specificity, *e.g.* biodifferental distribution, such as in myocardial tissue when the complexes of the invention are lipophilic in nature.

The details of one or more embodiments of the invention have been set forth in the accompanying description above. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs. All patents and publications cited in this specification are incorporated by reference.

The following Examples are presented in order to more fully illustrate the preferred embodiments of the invention. These Examples do not limit the scope of the invention, as defined by the appended claims.

### EXAMPLE 1

The thermodynamic stability constant of the Gd(III) complex of 3,6,10-tri(carboxymethly)-3,6,10-triazadodecanedioic acid (*e.g.*, the ligand of Formula I, without the benzyl-Z linker group), determined by potentiometry is logβ = 22.77 (Wang et al, Dalton, 1998, 41131). For a DTPA analogue complexes, it is well-known that groups attached to the DTPA backbone do not interfere in stability (e.g. EOB-DTPA and DTPA). By analogy, for the Gd(III)(EPTPA-bz-NO₂) complex (the ligand according to Formula I, where Z is a nitro group) one can also expect similar stability constant as the logβ determined for Gd(III)(EPTPA). This high stability ensures a very low toxicity (comparable to that of GdDTPA).

The Gd(EPTPA-bz-NO₂) complex (Formula I, Z is NO₂) has been investigated by ¹⁷O NMR, EPR and ¹H NMRD. According to the ¹⁷O chemical shifts, the complex has one inner sphere water molecule. This result is supported by UV-Vis measurements performed on the Eu(III) complex. The water exchange rate obtained is kₑₓ²⁹⁸ = 1.4 x 10⁸ s⁻¹, 40 times higher than that measured on Gd(DTPA).

In order to attain very high proton relaxivities for Gd(III) complexes, one has to increase the rotational correlation time of the molecule and optimise water exchange rate. Whereas increasing the rotational correlation time is relatively easy with the application of macromolecules, it is more difficult to fine-tune the water exchange rate without reducing the thermodynamic stability of the complex. Consequently, proton relaxivities of macromolecular agents are very often limited by slow water exchange.

The water exchange rate obtained for Gd(EPTPA-bz-NO₂) is in the optimal range to attain high proton relaxivities. This property, together with the high thermodynamic stability, is a unique feature among Gd(III) chelates known so far and it makes Gd(EPTPA-bz-NO₂) an ideal synthon to be attached to any rigid, slowly tumbling macromolecular agent. Coupling this chelate to a macromolecule with a rotational correlation time of 30 ns (that of serum albumin e.g.) should attain relaxivities of 50 mM⁻¹ s⁻¹ at 60 MHz proton Larmor frequency, although the monomer itself does not have high relaxivities. The proton relaxivities are shown in Table 1.

**Table 1:**

| **Proton relaxivities (mM**^{**-1**} **s**^{**-1**}**) of Gd(EPTPA-bz-NO**_{**2**}**) as a function of the proton Larmor frequency.** | | |
|---|---|---|
| **ν(MHz)** | **Relaxivity** | |
| | **24.8 °C** | **37.3 °C** |
| 9.998 | 5.563 | 4.428 |
| 5.999 | 6.339 | 5.216 |
| 3.598 | 7.235 | 5.671 |
| 2.159 | 7.415 | 5.803 |
| 0.778 | 8.296 | 6.197 |
| 0.778 | 8.390 | 6.368 |
| 0.467 | 8.214 | 6.490 |
| 0.280 | 8.558 | 6.573 |
| 0.168 | 8.474 | 6.550 |
| 0.101 | 8.348 | 6.903 |
| 0.061 | 8.691 | 6.449 |
| 0.036 | 8.635 | 6.560 |
| 0.022 | 8.543 | 6.478 |
| 12.001 | 5.475 | 4.187 |
| 14.002 | 5.154 | 4.205 |
| 16.000 | 4.988 | 4.261 |
| 18.002 | 4.911 | 4.129 |
| 20.001 | 4.730 | |

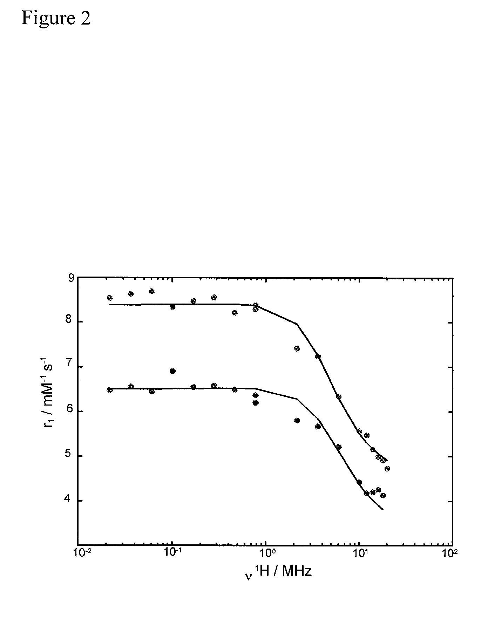

This information is presented graphically in Figure 2, which shows the NMRD profiles of Gd(EPTPA-bz-NO₂) at 37 °C (bottom curve) and 25°C (top curve).

The peak-to-peak line-widths of the EPR band of Gd(EPTPA-bz-NO₂) at 0.34 T measured as a function of temperature are shown in Table 2.

**Table 2:**

| **Peak-to-peak line widths of the EPR band of Gd(EPTPA-bz-NO**_{**2**}**) at 0.34 T measured as a function of the temperature.** | |
|---|---|
| **T (°C)** | **ΔH**_{**pp**} |
| 23.5 | 297.4 |
| 34.1 | 286 |
| 40.3 | 280.2 |
| 56.3 | 297.4 |
| 72.4 | 326 |
| 47.6 | 283.15 |
| 3 | 363.25 |

The variable temperature ¹⁷O NMR relaxation rates and chemical shifts are shown on Table 3.

**Table 3:**

| **Variable temperature** ^{**17**}**O NMR relaxation rates and chemical shifts.** | | | | | | | | | |
|---|---|---|---|---|---|---|---|---|---|
| **GdEPTPA-bz-NO**_{**2**} (C = 55.79 mM, pH = 6.0, référence = HClO₄) | | | | | | | | | |
| **T/K** | **1000/T/ K**^{**-1**} | **T**_{**1**}**/s(ref)** | **T**_{**1**}**/s** | **T**_{**2**}**/s(ref)** | **T**_{**2**}**/s** | **ν / Hz (ref.)** | **ν /Hz** | **Δw /Hz** | **Δw**_{**r**} **/Hz** |
| 359.7 | 2.78 | 2.17E-02 | 1.89E-02 | 2.23E-02 | 9.67E-03 | -3707.2 | -3803.0 | -95.8 | -5.99E+05 |
| 338.7 | 2.95 | 1.58E-02 | 1.37E-02 | 1.77E-02 | 6.16E-03 | -3664.8 | -3764.2 | -99.4 | -6.22E+05 |
| 324.2 | 3.08 | 1.22E-02 | 1.04E-02 | 1.38E-02 | 4.25E-03 | -3630.3 | -3751.5 | -121.2 | -7.58E+05 |
| 306.7 | 3.26 | 8.69E-03 | 7.34E-03 | 9.21E-03 | 2.63E-03 | -3582.3 | -3702.7 | -120.4 | -7.53E+05 |
| 298.0 | 3.36 | 7.07E-03 | 5.99E-03 | 9.21E-03 | 2.63E-03 | -3582.2 | -3705.7 | -123.5 | -7.73E+05 |
| 293.5 | 3.40 | 6.10E-03 | 5.13E-03 | 7.14E-03 | 1.98E-03 | -3565.2 | -3690.4 | -125.25 | -7.84E+05 |
| 286.7 | 3.49 | 5.07E-03 | 4.28E-03 | 6.17E-03 | 1.67E-03 | -3560.6 | -3686.0 | -125.36 | -7.84E+05 |
| 274.5 | 3.64 | 3.56E-03 | 3.01E-03 | 5.17E-03 | 1.35E-03 | -3545.5 | -3678.6 | -133.1 | -8.33E+05 |

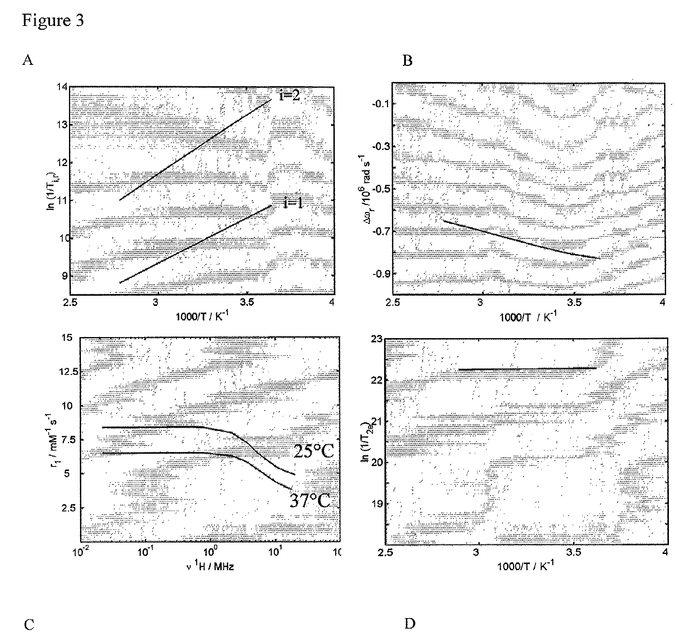

The ¹⁷O NMR, NMRD and EPR experimental data (points) and the fitted curves (lines) as obtained in a simultaneous analysis, according to the method of Powell et al., J. Am. Chem. Soc. 1996, 9333 are shown in Figure 3: 3A Top left: reduced transverse (i = 2) and longitudinal (i=1) ¹⁷O relaxation rates (B = 9.4 T), 3B: Top right: reduced ¹⁷O chemical shifts (B = 9.4 T). 3C: Bottom left: NMRD profiles. 3D: Bottom right: transverse electron spin relaxation rates at 0.34 T, measured by EPR.

The parameters obtained in the simultaneous analysis of ¹⁷O NMR, EPR and NMRD data for Gd(EPTPA-bz-NO₂) and Gd(DTPA) are shown in Table 4.

**Table 4:**

| Complexe de Gd(III) | DTPA | EPTPA-bz-NO₂ |
|---|---|---|
| Δ*H*^{‡} / kJ mol⁻¹ | 51.6 ± 1.4 | 23.7 ± 1.8 |
| Δ*S*^{‡} / J mol⁻¹K⁻¹ | 53.0 ± 4.7 | -9.2 ± 4.0 |
| ***k***_{**ex**}^{**298**} **/10**^{**6**} **s**^{**-1**} | **3.3 ± 0.2** | **146 ± 23** |
| *E*_{*r*} / kJ mol⁻¹ | 17.3 ± 0.8 | 19.9 ± 1.7 |
| τᵣ / ps | 58 ± 11 | 84.6 ± 6 |
| *E*ᵥ / kJ mol⁻¹ | 1.6 ± 1.8 | 1.0 |
| τᵥ/ps | 25 ± 1 | 20 ± 1 |
| Δ² /10²⁰ s⁻¹ | 0.46 ± 0.02 | 0.37 ± 0.03 |
| *A*/^{η}¤/10⁶ rad s⁻¹ | -3.8 ± 0.2 | -3.5 ± 0.2 |

The foregoing description has been presented only for the purposes of illustration and is not intended to limit the invention to the precise form disclosed, but by the claims appended hereto.

## Claims

1. A compound of Formula III 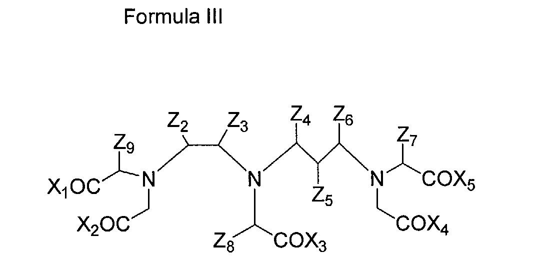 or a base or acid addition salt, hydrate, ester, solvate, prodrug, metabolite, stereoisomer, or mixture thereof, wherein at least one of Z₂ Z_{3,} Z_{4,} Z_{5,} Z_{6,} Z_{7,} Z_{8,} or Z₉ is a functionality suitable for coupling with a biocompatible macromolecule and each of the remaining Z₂ Z_{3,} Z_{4,} Z_{5,} Z_{6,} Z_{7,} Z_{8,} or Z₉ is a non-coordinating substituent;
X_{1,} X_{2,} X_{3,} X₄ and X₅ are independently OH or NR₂ wherein each R is a non-coordinating substituent;
with the proviso that at least two of X_{1,} X_{2,} X_{3,} X₄ and X₅ are OH.

2. The compound of claim 1, wherein each wherein each non coordinating substituent is, independently, a hydrogen.

3. The compound of claim 1, wherein each wherein each R is, independently, a hydrogen.

4. The compound of claim 1, wherein the functionality suitable for coupling with a biocompatible macromolecule is a isothiocyanato-benzyl.

5. The compound of claim 1, wherein the base or acid addition salt is a pharmaceutically acceptable salt.

6. A complex between a compound of claim 1 and a metal.

7. The complex of claim 6, wherein the metal is selected from the group consisting of elements having atomic numbers 21-29, 42-44, and 57-83.

8. The complex of claim 6, wherein the metal is selected from the group consisting of di- and tri-positive metals having a coordination number from 2 to 9.

9. The complex of claim 8, wherein the metal is selected from the group consisting of tri-positive metals having a coordination number of 8 or 9.

10. The complex of claim 9, wherein the metal is selected from the group consisting of Lanthanum, Europium, Gadolinium, Terbium, and Lutetium.

11. The complex of claim 6, wherein the metal is Gadolinium (III).

12. A compound of Formula II: 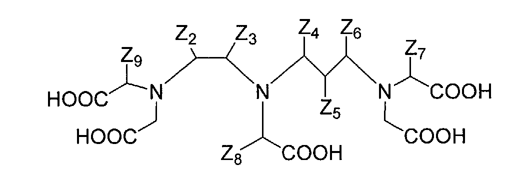 or a base or acid addition salt, hydrate, ester, solvate, prodrug, metabolite, stereoisomer, or mixture thereof, wherein at least one of Z₂ Z_{3,} Z_{4,} Z_{5,} Z_{6,} Z_{7,} Z_{8,} or Z₉ is a functionality suitable for coupling with a biocompatible macromolecule and each of the remaining Z₂ Z_{3,} Z_{4,} Z_{5,} Z_{6,} Z_{7,} Z_{8,} or Z₉ is a non-coordinating substituent.

13. The compound of claim 12, wherein each wherein each non coordinating substituent is, independently, a hydrogen.

14. The compound of claim 12, wherein the functionality suitable for coupling with a biocompatible macromolecule is a isothiocyanato-benzyl.

15. The compound of claim 12, wherein the base or acid addition salt is a pharmaceutically acceptable salt.

16. A complex between a compound of claim 12 and a metal.

17. The complex of claim 16, wherein the metal is selected from the group consisting of elements having atomic numbers 21-29, 42-44, and 57-83.

18. The complex of claim 17, wherein the metal is selected from the group consisting of di- and tri-positive metals having a coordination number from 2 to 9.

19. The complex of claim 18, wherein the metal is selected from the group consisting of tri-positive metals having a coordination number of 8 or 9.

20. The complex of claim 19, wherein the metal is selected from the group consisting of Lanthanum, Europium, Gadolinium, Terbium, and Lutetium.

21. The complex of claim 16, wherein the metal is Gadolinium (III).

22. The complex of claim 16 wherein Z is attached to a biocompatible macromolecule.

23. The complex of claim 22 wherein the biological molecule is a protein.

24. A method of magnetic resonance imaging a subject, the method comprising administering a complex of claim 6 and a non-toxic, pharmaceutically acceptable carrier, adjuvant, or other vehicle, and generating a magnetic resonance image of at least a part of said subject.

25. The method of claim 24, wherein the subject is a human patient.

26. A method of imaging a subject comprising the steps of (a) administering a contrast medium comprising a physiologically compatible complex of a ligand of III: and an element selected from the group consisting of metals having atomic numbers 21-29, 42-44, and 57-83; and (b) obtaining an image of said patient.

27. The method of claim 26, wherein the imaging is magnetic resonance imaging.

28. The method of claim 27, wherein the element is selected from the group consisting of the Lanthanides.

29. The method of claim 28, wherein the element is Gadolinium.

30. The method of claim 29, wherein the subject is human.

31. The method of claim 26, wherein the imaging is X-ray.

32. The method of claim 31, wherein the element is Bismuth.

33. The method of claim 26, wherein the imaging is ultrasound imaging.

34. The method of claim 26, wherein the imaging is scintigraphic imaging.

35. A method of radioimmunotherapy of a human subject applying a complex of claim 6, wherein the biocompatible macromolecule is a protein and the metal is selected from the group consisting of elements having atomic numbers 26, 27, 29, 31, 32, 37-39, 43, 49, 62, 64, 70, 75, 77, 82 and 83.

36. A compound of Formula I: 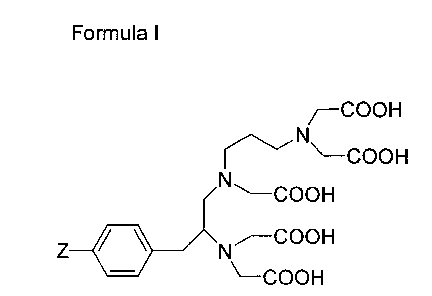 or a base or acid addition salt, hydrate, ester, solvate, prodrug, metabolite, stereoisomer, or mixture thereof, wherein Z is a hydrogen or a functionality suitable for coupling with a biocompatible macromolecule.

37. The compound of claim 36, wherein Z is selected from the group consisting of hydrogen, nitro and isothiocyanate.

38. The compound of claim 36, wherein the functionality suitable for coupling with a biocompatible macromolecule is a isothiocyanato-benzyl.

39. The compound of claim 36, wherein the base or acid addition salt is a pharmaceutically acceptable salt.

40. A complex between a compound of claim 36 and a metal.

41. The complex of claim 40, wherein the metal is selected from the group consisting of elements having atomic numbers 21-29, 42-44, and 57-83.

42. The complex of claim 41, wherein the metal is selected from the group consisting of di- and tri-positive metals having a coordination number from 2 to 9.

43. The complex of claim 36, wherein the metal is selected from the group consisting of tri-positive metals having a coordination number of 8 or 9.

44. The complex of claim 36, wherein the metal is selected from the group consisting of Lanthanum, Europium, Gadolinium, Terbium, and Lutetium.

45. The complex of claim 36, wherein the metal is Gadolinium (III).

46. A complex comprising Gadolinium (III) and at least one ligand of formula I:

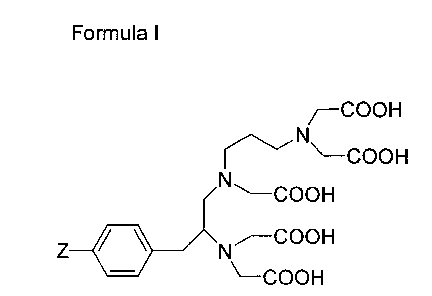

47. The complex of claim 46 wherein Z is attached to a biological molecule.

48. The complex of claim 47 wherein the biologocal molecule is a protein.

49. A method of magnetic resonance imaging a subject, the method comprising administering a complex of claim 48 and a non-toxic, pharmaceutically acceptable carrier, adjuvant, or other vehicle, and generating a magnetic resonance image of at least a part of said subject.

50. The method of claim 49, wherein the subject is a human patient.

51. A method of imaging a subject comprising the steps of (a) administering a contrast medium comprising a physiologically compatible complex of a ligand of Formula I: 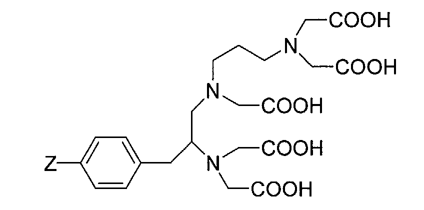 and an element selected from the group consisting of metals having atomic numbers 21-29, 42-44, and 57-83; and (b) obtaining an image of said patient.

52. The method of claim 51, wherein the imaging is magnetic resonance imaging.

53. The method of claim 52, wherein the element is selected from the group consisting of the Lanthanides.

54. The method of claim 53, wherein the element is Gadolinium.

55. The method of claim 54, wherein the subject is human.

56. The method of claim 51, wherein the imaging is X-ray.

57. The method of claim 56, wherein the element is Bismuth.

58. The method of claim 51, wherein the imaging is ultrasound imaging.

59. The method of claim 51, wherein the imaging is scintigraphic imaging.
